# EUROPEAN PATENT SPECIFICATION

(11) **EP 4 553 209 B1**
(45) Date of publication and mention of the grant of the patent: **04.11.2026**
(21) Application number: 25167259.8
(22) Date of filing: 15.06.2021
(51) Int. Cl.: D04B 21/10, D04B 21/12, A61F 2/00

(54) **PROSTHETIC REPAIR FABRIC**
PROTHETISCHES REPARATURGEWEBE
TISSU DE RÉPARATION PROTHÉTIQUE

(30) Priority: 19.06.2020 US 202016907104
(43) Date of publication of application: 14.05.2025
(62) Document number(s) of the earlier application(s) in accordance with Art. 76 EPC: 21758185.9 / 4 168 613
(73) Proprietor: Davol Inc., Warwick, RI 02886 (US)
(72) Inventor: FELIX, Augustus, Cranston, RI, 02921 (US); LIGEIKIS, Michael, Marlton, NJ, 08053 (US)
(74) Representative: Hoffmann Eitle

(56) References cited:
- US-A1- 2010 049 222
- US-B2- 9 416 471

## Description

### FIELD OF DISCLOSURE

The present disclosure relates to a prosthetic repair fabric, and more particularly to a prosthetic repair fabric for use in soft tissue and muscle wall repair.

### BACKGROUND

Implantable repair fabrics are employed by surgeons for soft tissue repair and reconstruction, including the repair of anatomical defects such as soft tissue and muscle wall defects. The fabric is typically sutured, stapled, tacked, or otherwise provisionally anchored in place over, under or within the defect. Tissue integration with the fabric, such as tissue ingrowth into and/or along the mesh fabric, eventually completes the repair.

Soft tissue and muscle wall defect repairs may be accomplished using various surgical techniques, including open, laparoscopic and hybrid (e.g., Kugel procedure) techniques. During open procedures, a repair fabric is placed through a relatively large incision made in the abdominal wall and layers of tissue and then the defect is filled or covered with the repair fabric. During laparoscopic and hybrid procedures, the fabric may be collapsed, such as by rolling or folding, into a reduced configuration for entry into a subject, either directly through a comparatively smaller incision or through a slender laparoscopic cannula that is placed through the incision.

Various repair fabrics are known and used for repairing soft tissue and muscle wall defects. BARD MESH and VISILEX, available from C.R Bard, and the repair fabric described in US 2010/0049222 A1 or US 9,416,471 B2 are examples of implantable fabrics that have been successfully used in soft tissue and muscle wall repair. Such fabrics are fabricated from polypropylene monofilaments that are knitted into meshes having pores or interstices that promote tissue ingrowth and integration with the fabric.

Scar tissue may form about a repair fabric into a scar plate as tissue ingrowth occurs. The volume and rigidity of the scar plate that forms about the fabric may be affected by various factors, including the amount of foreign material introduced into a patient by the fabric.

It is an object of the disclosure to provide a prosthetic repair fabric for repair of soft tissue and muscle wall defects.

### SUMMARY

According to an aspect of the present disclosure, there is provided an implantable prosthetic repair fabric, comprising: a biologically compatible, implantable dual bar warp knit mesh produced according to a first bar pattern chain of 4/2 4/6 4/2 6/8 6/4 6/8 and a second bar pattern chain of 6/8 2/0 6/8 4/2 8/10 4/2, the mesh is knitted of a first monofilament having a first diameter and a second monofilament having a second diameter which is greater than the first diameter, the first monofilament being knitted according to the first bar pattern chain and the second monofilament being knitted according to the second bar pattern chain.

### DESCRIPTION OF THE DRAWINGS

Various embodiments of the disclosure will now be described, by way of example, with reference to the accompanying drawings, in which:
FIG. 1 is an enlarged plan view of a dual bar warp knit, mesh fabric according to an illustrative embodiment of the present disclosure;
FIGS. 2A-2B illustrate the chain lapping pattern for the mesh fabric of FIG. 1; and
FIG. 3 is a schematic illustration for determining the area of a primary cell.

### DETAILED DESCRIPTION

Embodiments of the present disclosure include a prosthetic fabric comprising a mesh fabric that is relatively flexible, thin and light weight and meets the performance and physical characteristics for soft tissue repair and reconstruction procedures. The surgical repair fabric may be used for reinforcing and closing soft tissue defects, and is particularly indicated for chest wall reconstruction and/or the repair of hernias, such as inguinal hernias. The mesh fabric is formed of a biologically compatible, flexible and strong implantable material.

The mesh fabric may employ a knit construction that provides relatively large openings or pores to ensure good visibility of the underlying anatomy without sacrificing mechanical properties of the mesh. The porous character of the fabric allows tissue infiltration to incorporate the prosthetic. The knitted fabric is sufficiently strong and structured to prevent or minimize potential pullout of anchoring fasteners, such as sutures, staples, tacks, and the like. The flexible repair fabric may promote an easy reduction in size for entry into the subject. In this manner, the flexible fabric may be collapsed into a slender configuration, such as a roll, which can be supported in, and advanced through, a narrow laparoscopic cannula for use in laparoscopic procedures.

The mesh fabric employs a relatively lighter weight, thinner, and/or more flexible fabric construction that may introduce less foreign body material into a patient as compared to other repair fabrics. The porous prosthetic repair fabric allows a prompt fibroblastic response through the interstices of the mesh, forming a secure fibrous/prosthetic layer. The fabric may promote a thinner and more compliant scar plate that may result in a relatively more comfortable soft tissue or muscle wall repair for a patient.

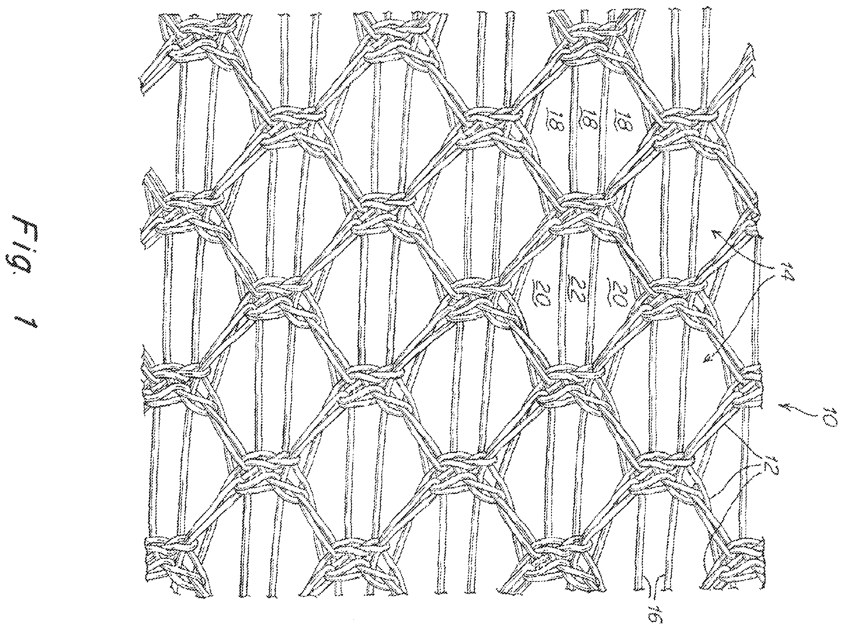

In one illustrative embodiment shown in FIG. 1, the repair fabric comprises a knit mesh 10 including knitted strands of filaments 12 that define larger, primary pores 14 arranged in a uniform pattern. A pair of individual filaments 16 extend across the primary pores to define a plurality of smaller, secondary pores 18 therein.

In the illustrated embodiment, the primary pores 14 are bounded by knitted strands of filaments 12. However, it is to be appreciated that one of more boundaries of the primary pores 14 may be defined by individual filaments as would be apparent to one of skill in the art. As shown, the primary pores 14 may have a generally polygon shape, such as hexagon, diamond or square shaped, although aspects of the disclosure are not limited. In this regard, it is to be understood that other pore shapes are also contemplated, including, but not limited to, circular, non-circular, round, oval and the like, as would be apparent to one of skill in the art.

The prosthetic repair fabric may be constructed to increase flexibility and/or reduce the overall weight per unit area of the fabric. Such properties may facilitate an easier collapse of the repair fabric for introduction into a patient. These properties may also provide for easier manipulation of the repair fabric about the surgical site within the patient. In one illustrative embodiment, the primary pores 14 have an area of approximately 0.0665 to 0.0794 cm² (0.01032 to 0.01233 square inches). In this regard, less material may be used to produce a given area of mesh, which may result in a reduced weight mesh. Additionally, the generally greater spacing between the strands of filaments 12 that are associated with the larger primary pores 14 may also contribute to a more flexible mesh. It is to be appreciated, however, that the size of the primary pores may vary as would be apparent to one of skill in the art, as aspects of the disclosure are not limited in this respect.

For some applications, it may be desirable to provide secondary pores 18 within the primary pores 14. In one illustrative embodiment shown in FIG. 1, each primary pore 14 is subdivided into a plurality of secondary pores 18 by a pair of individual or single filaments 16. In the illustrative embodiment, the pair of filaments 16 divides the primary pore 14 into a pair of generally triangular secondary 20 pores and a generally rectangular secondary pore 22 that is positioned between the two generally triangular secondary pores 20. It is to be appreciated, however, that the shapes of secondary pores and/or numbers of secondary pores within each primary pore, if desired, may vary as would be apparent to one of skill in the art, as aspects of the disclosure are not limited in this respect.

In one illustrative embodiment as shown in FIG. 1, the pair of individual filaments 16 extend substantially parallel to one another across the primary pores 14. As illustrated, the pair of parallel filaments 16 may be generally in linear alignment with corresponding pairs of filaments in adjacent primary pores. However, it is to be understood that the individual filaments may be positioned and oriented in other suitable arrangements within the scope of the claims.

The prosthetic repair fabric may be constructed so as to be provisionally anchored to tissue or muscle using a wide variety of fasteners, such as sutures, staples, spiral tacks, Q-rings and the like. The individual filaments 16 that extend across the primary pores may provide additional features for engaging the fasteners used to anchor the fabric. It is to be appreciated that repair fabrics may be anchored to tissue and/or mesh with fasteners, such as spiral tacks and Q-ring constructs, that have relatively small features for engaging and holding the repair fabric in place. The smaller, secondary pores 18 associated with the individual filaments may provide for improved engagement with the fasteners in a manner that is sufficiently strong and structured to prevent or minimize pullout. It is to be appreciated that the size of the secondary pores may vary as would be apparent to one of skill in the art, as aspects of the disclosure are not limited in this respect.

In one illustrative embodiment, the mesh fabric includes first filaments 12 having a first diameter to form the primary pores and second filaments 16 having a second diameter that is different from the first diameter extending across the primary pores. The second filaments 16 have a second diameter that is greater than the first diameter of the first filaments. Such an arrangement may enhance the handling of the mesh fabric by increasing its stiffness.

In one illustrative embodiment, the knit mesh 10 may be produced in a lapping pattern by using two partially threaded guide bars to knit the pattern over three needles in a six course repeat. The fabric structure may be of an atlas type where each knitted end travels more than two needles, which may prevent unraveling of the mesh.

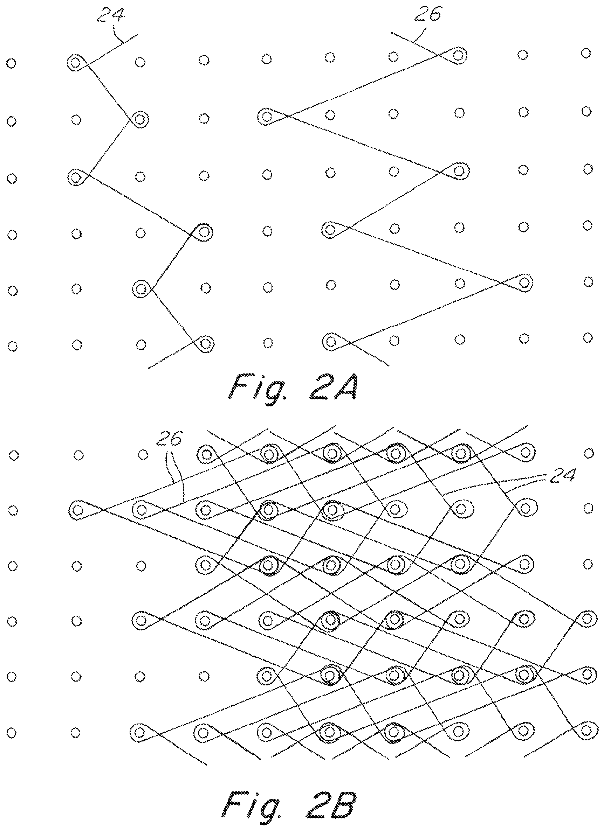

In one illustrative embodiment shown in FIG. 2, the repair fabric may employ a dual bar warp knit mesh structure produced using two guide bars moving according to a first bar pattern chain of 4/2 4/6 4/2 6/8 6/4 6/8 (identified as reference 24) and a second bar pattern chain of 6/8 2/0 6/8 4/2 8/10 4/2 (identified as reference 26). The mesh may be knitted on a single needle bar, 24 gauge Rachelle knitting machine. The mesh may be fabricated with approximately 34 to 36 courses per 25.4 mm (34 to 36 courses per inch) and approximately 12 to 17 wales per 25.4 mm (12 to 17 wales per inch). It is to be appreciated, however, that the mesh fabric may be knitted using any suitable knit pattern as would be apparent to one of skill in the art, as aspects of the disclosure are not limited in this respect.

The knit mesh may be produced at various widths apparent to one of skill in the art, such as from 2.54 cm to 203.2 cm ( 1 inch to 80 inches), depending on the intended application for which the repair fabric is being produced.

Following knitting, the fabric may be washed to remove foreign matter, such as residual processing lubricant. A cleaning agent, such as Triton X-100, may be used to aid in the removal of such foreign matter. Following washing, the mesh may be dried at a temperature lower than the heat set and melt temperatures of the material, as would be apparent to one of skill in the art.

Embodiments of the knit mesh may be heat set to impart a shape memory to the mesh and the prosthetic fabric formed of the mesh. In one illustrative embodiment, the fabric is heat set to have a generally planar shape memory. In this manner, after the fabric is collapsed and inserted into a patient, the fabric may revert back to the planar configuration for appropriate placement against tissue of the patient. It is to be appreciated that other embodiments of the fabric may be provided with a shape memory that corresponds to configurations different than planar, or to have no shape memory at all, as aspects of the disclosure are not limited in this regard.

If desired, the knit mesh may be heat set under tension, in a crochet hoop or tentering frame. The heat set may be applied while the mesh knit is being stretched in a particular direction to help set the mesh into a particular configuration. In one illustrative embodiment, the knit mesh is stretched in the cross machine knit direction and simultaneously allowed to partially relax or contract in the machine direction to a fixed point while heat is applied to set the mesh. It is to be understood, however, that other techniques apparent to one of skill in the art may be used to heat set the knit mesh, as aspects of the disclosure are not limited in this respect.

For some applications, it may be desirable to smooth the knitted mesh to reduce the texture or surface roughness of the mesh. In one illustrative embodiment, the knitted mesh is lightly pressed between a pair of plates which includes a heated plate that is pressed against the rough surface of the mesh to reduce high spots of the mesh and to heat set it to smooth its surface. It is to be appreciated, however, that the fabric may be smoothed using any suitable process apparent to one of skill in the art. For example, the fabric may be smoothed by passing the knitted mesh between a pair of heated rollers during the washing and drying process.

The filaments that are used to fabricate the repair fabric may contribute to the resulting mechanical properties of the fabric. In one illustrative embodiment, the repair fabric is knitted with first filaments 12 having a diameter of approximately 0.1143 to 0.1295 mm (0.0045 to 0.0051 inches) (first bar pattern chain), and preferably a diameter of approximately 0.1219 mm (0.0048 inches), and second filaments 16 having a diameter of approximately 0.0063 to 0.1905 mm (0.0063 to 0.0075 inches) (second bar pattern chain), and preferably a diameter of approximately 0.1905 mm (0.0075 inches). Filaments of these diameters may contribute to an enhanced handling and increased strength properties of the overall repair fabric.

In one illustrative embodiment, the fabric has a thickness of approximately 0.589 to 0.610 mm (0.022 to 0.024 inches), and preferably a thickness of approximately 5.715 to 5.969 mm (0.0225 to 0.0235 inches). In one illustrative embodiment, the fabric has a weight per unit area of approximately 0.010 to 0.011 grams/cm² (0.066 to 0.069 grams per square inch). It is to be appreciated, however, that the fabric may be fabricated to have any thickness and/or weight per unit area apparent to one of skill in the art that is suitable for a desired application, as aspects of the disclosure are not limited in this respect.

In one illustrative embodiment, the filaments used to fabricate the mesh fabric comprise a polypropylene monofilament, which is inert in the presence of infection, is non-wettable and has a low foreign body reaction. In one illustrative embodiment, the monofilaments are formed of Aran Biomedical ProTex Med Polypropylene resin PPS50156 and PPS50157. In one embodiment, the first monofilament has a denier of approximately 98±11 (10.89±1.22 tex) and the second monofilament has a denier of approximately 240±20 (26.67±2.22 tex). In one embodiment, the first and second monofilaments have a tenacity of approximately 5.4 to 7.65 grams/decitex (6.0 to 8.5 grams/denier), with a nominal tenacity of approximately 5.58 grams/decitex (6.2 grams/denier). It is to be appreciated, however, that filaments of different configurations, properties and/or materials may be employed to fabricate the fabric within the scope of the claims.

For example, the filaments may comprise multifilaments or monofilaments having different mechanical characteristics as would be apparent to one of skill in the art, within the scope of the claims.

### EXAMPLES

The following examples are illustrative only and are not intended to limit the scope of the present disclosure.

Physical properties of a representative two bar warp knit mesh fabric produced from 0.1219 mm (0.0048 inch) (first bar) and 0.1905 mm (0.0075 inch) (second bar) polypropylene monofilament according to the illustrative embodiment shown in FIGS. 1 and 2 (labeled Embodiment #1 in Table 1) were evaluated and compared to several known mesh fabrics (comparative mesh fabrics). Physical and performance characteristics were tested including mesh thickness, pore size, mesh weight per unit area, suture pull out strength, burst strength, tear resistance, tensile (break) strength and elongation at break, and stiffness. Testing methodology and results appear below in Table 1, where mean results and ranges are reported from several test samples (ranges appear in parentheses).

**Suture Pullout Strength:** A sample of mesh measuring at least 25.4 mm x 25.4 mm (1 inch x 1 inch) (Embodiment #1) or at least 12.7 mm x 76.2 mm (0.5 inch x 3 inches) (comparative mesh fabrics) was prepared and clamped in the lower jaw of an MTS^{™} or equivalent tensile test machine. The long dimension of the sample should be parallel with its orientation designation (machine or cross-machine). At least 12.7 mm (0.5 inch) (Embodiment #1) or at least 25.4 mm (1 inch) (comparative mesh fabrics) of the mesh was exposed above the jaw. A spring steel wire with a diameter of approximately 0.482 mm (0.019 inches) was placed through the mesh to simulate a suture. The wire was placed 5±1mm from the edge of the mesh. The wire suture was looped back and both ends were attached to the upper jaw of the tensile machine. The suture was then pulled at a rate of 127 mm (5 inches) per minute through the mesh. The peak force was recorded for 4 to 10 samples tested in both the machine and cross directions of the mesh and the average force was calculated for at least 10 total measurements in each direction.

**Pore Size:** A sample of mesh was placed on an optical coordinate measurement device such as a Tesa Vision (35x) or equivalent.

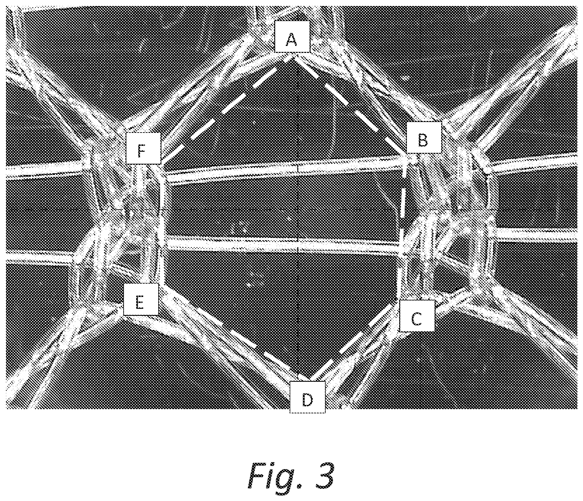

For embodiment#1, each primary pore has a generally hexagon shape which contains two generally triangular pores and a generally rectangular pore in the middle section. The length L of each leg of the primary pore was measured dimensionally between each pair of end points A-B, B-C, C-D, D-E, E-F and F-A, as illustrated by dashed lines in FIG. 3. The pore area of the primary pore was calculated based on the area of a hexagon as follows, where L_{average} is the average length of each leg: $Area = {\left({L}_{average}\right)}^{2} \times \left(3√3\right) / 2$ Three randomly selected primary cells (not counting the pores formed by the loops or knots) of each of four mesh samples were measured and a combined average was calculated.

**Tensile (Break) Strength and Elongation at Break:** A mesh sample measuring approximately 25.4 mm x 152 mm (1 inch x 6 inches) was placed into the pneumatic jaws of an MTS^{™} tensile tester or equivalent device. The sample was oriented so that the knit direction being tested was parallel to the 152 mm (6 inch) length. The ends of the 152 mm (6 inch) sample were gripped in the lower and upper jaws of the tester. Starting with a minimum separation of 50.8 mm (2 inches), the sample was pulled at a constant rate of 304.8 mm (12 inches) per minute until the sample broke. The peak load and elongation at break were recorded. The samples were tested in both the cross direction and the machine direction. The averages of at least 10 total measurements taken from 4 to 10 samples were then calculated for each direction.

**Mesh Thickness:** A sample of mesh was measured using a standard thickness snap gage with an approximate 9.65 mm (0.38 inch) diameter pressure foot that is lightly spring loaded. The thickness was measured by lowering the foot onto the mesh. Measurements were taken to the nearest 0.0025 mm (0.0001 inch). At least five sheets of mesh were measured in total and a combined average was calculated.

**Mesh Weight/Unit** Area: Using a sample size of at least four pieces of mesh that measured at least approximately 50.8 mm x 50.8 mm (2 inches x 2 inches), the weight of each sample was measured in grams to the nearest 0.0001 gram. The area was calculated by measuring the length and width dimensions taken to the nearest 0.025 mm (0.001 inch), minus the area of any radiused corner. The weight per unit area was calculated for each sample using the weight and unit area. The average weight per unit area was calculated by combining and averaging the weight per unit area for each sample.

**Burst Strength:** This test method was derived from the ANSI/AAMI VP20-1994 Section 8.3.3.2 and ASTM Ball Burst method D3787-01. A mesh sample was placed on top of a circular O-ring measuring approximately 25.4 mm (1 inch) in diameter. The O-ring was seated in a grooved plate in a fixture with a hole in the middle of plate containing the O-ring. The fixture was attached to the lower jaw in an MTS^{™} or equivalent test machine. The plate with the mesh was raised and clamped against an upper plate in the fixture, compressing the mesh sample. The upper plate also contained a hole with the same diameter as the lower plate. The holes in the fixture plates are dimensioned to be just slightly larger than and to accept a rounded ball tipped rod that has a 9.65 mm (0.38 inch) diameter tip. The rod was connected to an upper jaw of the test machine that was moved down through the sample at a constant rate of 304.8 mm (12 inches) per minute. The peak load was recorded for at least 10 samples. The average burst strength was then calculated based on the peak loads for the samples.

**Tear Resistance:** A mesh sample measuring approximately 50.8 mm x 50.8 mm (2 inches x 2 inches) was prepared. A 25.4 mm (1 inch) slit was cut in one side (the direction to be tested) at the midpoint to form two mesh sections. One section of mesh was clamped in the lower jaw of a pneumatic fixture and the other was clamped in the top jaw of the fixture. Starting with the jaws at a minimum spacing of 25.4 mm (1 inch), the mesh was pulled at a rate of 304.8 mm (12 inches) per minute until the tear was completed. The peak force was recorded. Samples were tested in the cross direction and the machine direction (Embodiment #1), and the cross direction, the machine direction, and the diagonal direction (comparative mesh fabrics). The averages of at least 10 total measurements taken from 4 to 10 samples were then calculated for each group direction.

**TABLE I**

| | | **Embodiment #1** | **BARD Soft Mesh** | **BARD MESH** | **Ethicon PROLENE Soft Mesh** | **Ethicon MERSILENE Mesh** |
|---|---|---|---|---|---|---|
| **Suture Pullout (lbs)** | | | | | | |
| | Machine Direction | 4.6kg (10.2) | 3.7 kg (8.2) | 5.5 kg (12.16) | 2.4 kg (5.4) | 0.79 kg (1.75) |
| | | 2.77-5.85 kg (6.1 to 12.9) | 3.22-4.35 kg (7.1 - 9.6) | 4.15-6.48 kg (9.14 - 14.29) | 1.77-2.99 kg (3.9 - 6.6) | 0.60-1.06 kg (1.33 - 2.33) |
| | Cross Direction | 3.86 kg (8.5) | 3.04 kg (6.7) | 3.62 kg (7.97) | 2.86 kg (6.3) | 0.98 kg (2.17) |
| | | 1.45-0.68 kg (3.2 - 15.0) | 2.18-3.63 kg (4.8 - 8.0) | 3.12-4.41 kg (6.87 - 9.73) | 2.35-3.63 kg (5.2 - 8.0) | 0.69-1.12 kg (1.53 - 2.47) |

| **Pore Size (inches²)** | | | | | | |
|---|---|---|---|---|---|---|
| | Large Cell | 0.07052 cm² (0.01093) | 0.06290 cm² (0.00975) | n/a | 0.06071 cm² (0.00941) | n/a |
| | | 0.06658-0.07955 cm² (0.01032 - 0.01233) | 0.05748-0.06665 cm² (0.00891 - 0.01033) | | 0.05181-0.0671 cm² (0.00803 - 0.0104) | |
| | Small Pore | n/a | 0.01587 cm² | 0.00548 cm² | cm² | 0.00794 cm² |
| | | | (0.00246) | (0.00085) | (0.00386) | (0.00123) |
| | | | 0.01419-0.02065 cm² (0.00220 - 0.00320) | 0.00400-0.0065 cm² (0.00062 - 0.00100) | 0.02303-0.02787 cm² (0.00357 - 0.00432) | 0.00671-0.00897 cm² (0.00104 - 0.00139) |

| **Tensile (Break) Strength (lbs)** | | | | | | |
|---|---|---|---|---|---|---|
| | Machine Direction | 363.2 kg (14.3) | 303.02 kg (11.93) | 551.18 kg (21.7) | 561.59 kg (22.11) | 576.6 kg (22.7) |
| | | 241.3 to 411.5 kg (9.5 to 16.2) | 255.78-386.08 kg (10.07 - 15.20) | 353.1-680.7 kg (13.9 - 26.8) | 457.45 to 680.7 kg (18.01 - 26.8) | 515.6-660.4 kg (20.3 - 26) |
| | Cross Direction | 1,033.8 kg (40.7) | 1,113.54 kg (43.84) | 1.267.5 kg (49.9) | 558.04 kg (21.97) | 266.7 kg (10.5) |
| | | 718.8-1,325.9 kg (28.3 to 52.2) | 895.60-1,228.85 kg (35.26 - 48.38) | 1,084.6-1,473.2 kg (42.7 - 58.0) | 482.60-660.65kg (19.00 - 26.01) | 226.1-304.8 kg (8.9 - 12) |

| **Elongation at Break (%)** | | | | | | |
|---|---|---|---|---|---|---|
| | Machine Direction | 77.4 (68 - 86.5) | 61.8 (26.0 - 94.5) | 43 (30 - 50) | 69.25 (59 - 79.5) | 24 (22 - 26) |
| | Cross Direction | 34.7 (20 - 40) | 49 (40.5 - 54) | 30 (25 - 35) | 54.5 (49.5 - 63.5) | 25 (21 - 29) |

| **Mesh Thickness (Inches)** | | | | | | |
|---|---|---|---|---|---|---|
| | | 0.5817mm (0.0229) | 0.4420mm (0.0174) | 0.7061mm (0.0278) | 0.4267mm (0.0168) | 0.5156mm (0.0203) |
| | | 0.5715-0.5969 mm (0.0225 - 0.0235) | 0.4267-0.4369 mm (0.0168 - 0.0172) | 0.6934-0.7518 mm (0.0273 - 0.0296) | 0.4140-0.4369 mm (0.0163 - 0.0172) | 0.5080-0.5182 mm (0.0200 - 0.0204) |

| **Weight/Unit Area** (gms/ **inches²)** | | | | | | |
|---|---|---|---|---|---|---|
| | | 104.0 g/m² (0.0671) | 43.7 g/m² (0.0282) | 105.4 g/m² (0.0680) | 444.9 g/m² (0.0287) | 42.47 g/m² (0.0274) |
| | | 102.5-107.0 g/m² (0.0661 - 0.0690) | 42.5-45.4 g/ₘ² (0.0274 - 0.0293) | 100.8-110.1 g/m² (0.065 - 0.071) | 42.3-45.9 g/ₘ² (0.0273 - 0.0296) | 40.3-46.5 g/ₘ² (0.026 - 0.030) |

| **Tear Resistance (lbs)** | | | | | | |
|---|---|---|---|---|---|---|
| | Machine Direction | 3.94 kg (8.69) | kg (6.36) | kg (10.31) | kg (6.05) | kg (1.53) |
| | | 3.34-4.69 kg (7.37 - 10.33) | 2.43-3.16 kg (5.35 - 6.96) | 3.30-6.66 kg (7.27 - 14.68) | 2.13-3.24 kg (4.70 - 7.15) | 0.60-0.85 kg (1.33 - 1.87) |
| | Cross Direction | 4.35 kg (9.60) | 2.48 kg (5.46) | 4.99 kg (11.01) | 2.59 kg (5.72) | 0.68 kg (1.50) |
| | | 3.35-4.96 kg (7.38 - 10.93) | 2.20-2.86 kg (4.84 - 6.30) | 4.27-6.21 kg (9.41 - 13.68) | 2.17-2.92 kg (4.78 - 6.43) | 0.60-0.79 kg (1.33 - 1.74) |
| | Diagonal Direction | n/a | 2.69 kg (5.94) | 4.91 kg (10.83) | 2.71 kg (5.97) | 0.65 kg (1.44) |
| | | | 2.35-3.09 kg (5.18 - 6.82) | 3.27-6.47 kg (7.20 - 14.26) | 2.39-3.02 kg (5.27 - 6.65) | 0.58-0.73 kg (1.27 - 1.60) |

| **Ball Burst (3/8" ball, lbs)** | | | | | | |
|---|---|---|---|---|---|---|
| | | 16.70 kg (36.82) | 13.23 kg (29.17) | 30.84 kg (67.98) | 14.07 kg (31.01) | 8.21 kg (18.1) |
| | | 12.36-19.22 kg (27.24 - 42.38) | 11.35-15.85 kg (25.02 - 34.94) | 28.87-33.66 kg (63.64 - 74.21) | 12.06-15.93 kg (26.58 - 35.13) | 7.26-8.80 kg (16.0 - 19.4) |
| **Mesh Construction** | | | | | | |
| | Courses (per 25.4 mm) (per inch) | 36 | 34.6 (34 - 36) | 54.5 | 45.6 (44 - 48) | 72 |
| | Wales (per 25.4 mm) (per inch) | 14 (13.5 - 14.5) | 14.1 (12-17) | 15.5 | 16.2 (16 - 18) | 47 |

It should be understood that the foregoing description of the disclosure is intended merely to be illustrative thereof and that embodiments and modifications of the disclosure are within the scope of the disclosure recited in the claims appended hereto.

## Claims

1. An implantable prosthetic repair fabric, comprising:
a biologically compatible, implantable dual bar warp knit mesh (10) produced according to a first bar pattern chain of 4/2 4/6 4/2 6/8 6/4 6/8 and a second bar pattern chain of 6/8 2/0 6/8 4/2 8/10 4/2, the mesh is knitted of a first monofilament (12) having a first diameter and a second monofilament (16) having a second diameter, the first monofilament being knitted according to the first bar pattern chain and the second monofilament being knitted according to the second bar pattern chain;
the implantable prosthetic repair fabric being **characterized in that** the second diameter is greater than the first diameter.

2. The implantable prosthetic repair fabric according to claim 1, wherein the first and second monofilaments (12, 16) include polypropylene monofilament;
optionally wherein the first monofilament (12) has a diameter of approximately 0.1143 to 0.1295 mm (0.0045 to 0.0051 inches) and the second monofilament (16) has a diameter of approximately 0.1600 to 0.1905 mm (0.0063 to 0.0075 inches); or
optionally the first monofilament (12) has a diameter of approximately 0.1219 mm (0.0048 inches) and the second monofilament (16) has a diameter of approximately 0.1905 mm (0.0075 inches); or
optionally the first monofilament (12) has a linear mass density of approximately 10.89 tex ± 1.22 tex ( denier of approximately 98±11 ) and the second monofilament (16) has a linear mass density of approximately 26.67 tex ± 2.22 tex ( denier of approximately 240±20); or
optionally the first and second monofilaments (12, 16) have a tenacity of approximately 5.4 to 7.65 grams per dtex ( approximately 6.00 to 8.50 grams per denier); or
optionally the first and second monofilaments (12, 16) have a tenacity of approximately 5.58 grams per dtex ( approximately 6.2 grams per denier).

3. The implantable prosthetic repair fabric according to claim 1, wherein the mesh (10) has a knit construction that includes approximately 34 to 36 courses per 25.4 mm (34 to 36 courses per inch) and approximately 12 to 17 wales per 25.4 mm (12 to 17 wales per inch); or
wherein the mesh (10) has a weight per unit area of approximately 0.0102 to 0.0107 grams/cm² (0.0661 to 0.0690 grams per square inch); or
wherein the mesh (10) has a thickness of approximately 0.589 to 0.610 mm (0.022 to 0.024 inches).

4. The implantable prosthetic repair fabric according to claim 1, wherein the mesh (10) has a ball burst strength of 15.88 kg to 19.23 kg (35 lbs to 42.4 lbs);
optionally wherein the mesh (10) has a ball burst strength of 16.33 kg to 18.14 kg (36 lbs to 40 lbs); or
optionally wherein the mesh (10) has a ball burst strength of 16.33 kg to 17.69 kg (36 lbs to 39 lbs).

5. The implantable prosthetic repair fabric according to claim 1, wherein the mesh (10) has a suture pullout strength of 4.08 kg to 4.99 kg (9 lbs to 11 lbs) in the machine direction and 3.40 kg to 4.31 kg (7.5 lbs to 9.5 lbs) in the cross direction;
optionally wherein the mesh (10) has a suture pullout strength of approximately 4.63 kg (10.2 lbs) in the machine direction and approximately 3.86 kg (8.5 lbs) in the cross direction.

6. The implantable prosthetic repair fabric according to claim 1, wherein the mesh (10) has a tensile strength of 5.35 kg to 7.62 kg (11.8 lbs to 16.8 lbs) in the machine direction and 15.51 kg to 21.41 kg (34.2 lbs to 47.2 lbs) in the cross direction;
optionally wherein the mesh (10) has a tensile strength of approximately 6.49 kg (14.3 lbs) in the machine direction and approximately 18.46 kg (40.7 lbs) in the cross direction.

7. The implantable prosthetic repair fabric according to claim 1,
wherein the mesh (10) has a ball burst strength of 15.88 kg to 19.23 kg (35 lbs to 42.4 lbs), a suture pullout strength of 4.08 kg to 4.99 kg (9 lbs to 11 lbs) in the machine direction and 3.40 kg to 4.31 kg (7.5 lbs to 9.5 lbs) in the cross direction, and a tensile strength of 5.35 kg to 7.62 kg (11.8 lbs to 16.8 lbs) in the machine direction and 15.51 kg to 21.41 kg (34.2 lbs to 47.2 lbs) in the cross direction.

8. The implantable prosthetic repair fabric according to claim 7, wherein the mesh (10) has a ball burst strength of 16.33 kg to 17.69 kg (36 lbs to 39 lbs).

9. The implantable prosthetic repair fabric according to claim 7, wherein the mesh (10) has a suture pullout strength of approximately 4.63 kg (10.2 lbs) in the machine direction and approximately 3.86 kg (8.5 lbs) in the cross direction.

10. The implantable prosthetic repair fabric according to claim 7, wherein the mesh (10) has a tensile strength of approximately 6.49 kg (14.3 lbs) in the machine direction and approximately 18.46 kg (40.7 lbs) in the cross direction.

11. The implantable prosthetic repair fabric according to claim 7, wherein the mesh (10) has a knit construction that includes approximately 34 to 36 courses per 25.4 mm (34 to 36 courses per inch) and approximately 12 to 17 wales per 25.4 mm (12 to 17 wales per inch).

12. The implantable prosthetic repair fabric according to claim 7, wherein the mesh (10) has a weight per unit area of approximately 0.0102 to 0.0107 grams/cm² (0.0661 to 0.0690 grams per square inch).

13. The implantable prosthetic repair fabric according to claim 7, wherein the mesh (10) has a thickness of approximately 0.589 to 0.610 mm (0.022 to 0.024 inches).

## Patentansprüche

1. Implantierbare Prothesenreparaturgewirke, umfassend:
ein biologisch verträgliches, implantierbares zweibarriges Kettenwirknetz (10), das gemäß einer ersten Legebarren-Legungsnotation von 4/2 4/6 4/2 6/8 6/4 6/8 und einer zweiten Legebarren-Legungsnotation von 6/8 2/0 6/8 4/2 8/10 4/2 hergestellt ist, wobei das Netz aus einem ersten Monofilament (12), das einen ersten Durchmesser aufweist, und einem zweiten Monofilament (16), das einen zweiten Durchmesser aufweist, gewirkt ist, wobei das erste Monofilament gemäß der ersten Legebarren-Legungsnotation gewirkt ist und das zweite Monofilament gemäß der zweiten Legebarren-Legungsnotation gewirkt ist; wobei das implantierbare Prothesenreparaturgewirke **dadurch gekennzeichnet ist, dass** der zweite Durchmesser größer ist als der erste Durchmesser.

2. Implantierbare Prothesenreparaturgewirke nach Anspruch 1, wobei das erste und zweite Monofilament (12, 16) Polypropylen-Monofilament einschließen;
wahlweise, wobei das erste Monofilament (12) einen Durchmesser von ungefähr 0,1143 bis 0,1295 mm (0,0045 bis 0,0051 Zoll) aufweist und das zweite Monofilament (16) einen Durchmesser von ungefähr 0,1600 bis 0,1905 mm (0,0063 bis 0,0075 Zoll) aufweist; oder
wahlweise, wobei das erste Monofilament (12) einen Durchmesser von ungefähr 0,1219 mm (0,0048 Zoll) aufweist und das zweite Monofilament (16) einen Durchmesser von ungefähr 0,1905 mm (0,0075 Zoll) aufweist; oder
wahlweise, wobei das erste Monofilament (12) eine lineare Massendichte von ungefähr 10,89 tex ± 1,22 tex (Denier von ungefähr 98±11) aufweist und das zweite Monofilament (16) eine lineare Massendichte von ungefähr 26,67 tex ± 2,22 tex (Denier von ungefähr 240±20) aufweist; oder
wahlweise, wobei das erste und das zweite Monofilament (12, 16) eine Tenazität von ungefähr 5,4 bis 7,65 Gramm pro dtex (ungefähr 6,00 bis 8,50 Gramm pro Denier) aufweisen; oder
wahlweise, wobei das erste und das zweite Monofilament (12, 16) eine Tenazität von ungefähr 5,58 Gramm pro dtex (ungefähr 6,2 Gramm pro Denier) aufweisen.

3. Implantierbare Prothesenreparaturgewirke nach Anspruch 1, wobei das Netz (10) eine Strickkonstruktion aufweist, die etwa 34 bis 36 Maschenreihen pro 25,4 mm (34 bis 36 Maschenreihen pro Zoll) und etwa 12 bis 17 Maschenstäbchen pro 25,4 mm (12 bis 17 Maschenstäbchen pro Zoll) einschließt; oder
wobei das Netz (10) ein Flächengewicht von ungefähr 0,0102 bis 0,0107 Gramm/cm² (0,0661 bis 0,0690 Gramm pro Quadratzoll) aufweist; oder
wobei das Netz (10) eine Dicke von etwa 0,589 bis 0,610 mm (0,022 bis 0,024 Zoll) aufweist.

4. Implantierbare Prothesenreparaturgewirke nach Anspruch 1, wobei das Netz (10) eine Kugelberstfestigkeit von 15,88 kg bis 19,23 kg (35 lbs bis 42,4 lbs) aufweist;
wahlweise, wobei das Netz (10) eine Kugelberstfestigkeit von 16,33 kg bis 18,14 kg (36 lbs bis 40 lbs) aufweist; oder
wahlweise, wobei das Netz (10) eine Kugelberstfestigkeit von 16,33 kg bis 17,69 kg (36 lbs bis 39 lbs) aufweist.

5. Implantierbare Prothesenreparaturgewirke nach Anspruch 1, wobei das Netz (10) eine Nahtausreißfestigkeit von 4,08 kg bis 4,99 kg (9 lbs bis 11 lbs) in Maschinenrichtung und 3,40 kg bis 4,31 kg (7,5 lbs bis 9,5 lbs) in Querrichtung aufweist;
wahlweise, wobei das Netz (10) eine Nahtausreißfestigkeit von ungefähr 4,63 kg (10,2 lbs) in der Maschinenrichtung und ungefähr 3,86 kg (8,5 lbs) in der Querrichtung aufweist.

6. Implantierbare Prothesenreparaturgewirke nach Anspruch 1, wobei das Netz (10) eine Zugfestigkeit von 5,35 kg bis 7,62 kg (11,8 lbs bis 16,8 lbs) in der Maschinenrichtung und 15,51 kg bis 21,41 kg (34,2 lbs bis 47,2 lbs) in der Querrichtung aufweist;
wahlweise, wobei das Netz (10) eine Zugfestigkeit von ungefähr 6,49 kg (14,3 lbs) in der Maschinenrichtung und ungefähr 18,46 kg (40,7 lbs) in der Querrichtung aufweist.

7. Implantierbare Prothesenreparaturgewirke nach Anspruch 1,
wobei das Netz (10) eine Kugelberstfestigkeit von 15,88 kg bis 19,23 kg (35 lbs bis 42,4 lbs) aufweist, eine Nahtausreißfestigkeit von 4,08 kg bis 4,99 kg (9 lbs bis 11 lbs) in der Maschinenrichtung und 3,40 kg bis 4,31 kg (7,5 lbs bis 9,5 lbs) in der Querrichtung aufweist und eine Zugfestigkeit von 5,35 kg bis 7,62 kg (11,8 lbs bis 16,8 lbs) in der Maschinenrichtung und 15,51 kg bis 21,41 kg (34,2 lbs bis 47,2 lbs) in der Querrichtung aufweist.

8. Implantierbare Prothesenreparaturgewirke nach Anspruch 7, wobei das Netz (10) eine Kugelberstfestigkeit von 16,33 kg bis 17,69 kg (36 lbs bis 39 lbs) aufweist.

9. Implantierbare Prothesenreparaturgewirke nach Anspruch 7, wobei das Netz (10) eine Nahtausreißfestigkeit von etwa 4,63 kg (10,2 lbs) in Maschinenrichtung und etwa 3,86 kg (8,5 lbs) in Querrichtung aufweist.

10. Implantierbare Prothesenreparaturgewirke nach Anspruch 7, wobei das Netz (10) eine Zugfestigkeit von etwa 6,49 kg (14,3 lbs) in Maschinenrichtung und etwa 18,46 kg (40,7 lbs) in Querrichtung aufweist.

11. Implantierbare Prothesenreparaturgewirke nach Anspruch 7, wobei das Netz (10) eine Maschenkonstruktion aufweist, die ungefähr 34 bis 36 Maschenreihen pro 25,4 mm (34 bis 36 Maschenreihen pro Zoll) und ungefähr 12 bis 17 Maschenstäbchen pro 25,4 mm (12 bis 17 Maschenstäbchen pro Zoll) einschließt.

12. Implantierbare Prothesenreparaturgewirke nach Anspruch 7, wobei das Netz (10) ein Flächengewicht von ungefähr 0,0102 bis 0,0107 Gramm/cm² (0,0661 bis 0,0690 Gramm pro Quadratzoll) aufweist.

13. Implantierbare Prothesenreparaturgewirke nach Anspruch 7, wobei das Netz (10) eine Dicke von etwa 0,589 bis 0,610 mm (0,022 bis 0,024 Zoll) aufweist.

## Revendications

1. Tissu de réparation prothétique implantable, comprenant :
une maille tricotée chaîne à deux barres (10) implantable, biologiquement compatible, produite selon une première chaîne de motifs de barres de 4/2 4/6 4/2 6/8 6/4 6/8 et une seconde chaîne de motifs de barres de 6/8 2/0 6/8 4/2 8/10 4/2, la maille étant tricotée à partir d'un premier monofilament (12) présentant un premier diamètre et d'un second monofilament (16) présentant un second diamètre, le premier monofilament étant tricoté selon la première chaîne de motifs de barres et le second monofilament étant tricoté selon la seconde chaîne de motifs de barres ; le tissu de réparation prothétique implantable étant **caractérisé en ce que** le second diamètre est supérieur au premier diamètre.

2. Tissu de réparation prothétique implantable selon la revendication 1, dans lequel les premier et second monofilaments (12, 16) incluent un monofilament de polypropylène ;
facultativement dans lequel le premier monofilament (12) présente un diamètre d'approximativement 0,1143 à 0,1295 mm (0,0045 à 0,0051 pouces) et le second monofilament (16) présente un diamètre d'approximativement 0,1600 à 0,1905 mm (0,0063 à 0,0075 pouces) ; ou
facultativement le premier monofilament (12) présente un diamètre d'approximativement 0,1219 mm (0,0048 pouces) et le second monofilament (16) présente un diamètre d'approximativement 0,1905 mm (0,0075 pouces) ; ou
facultativement le premier monofilament (12) présente une masse linéique d'approximativement 10,89 tex ± 1,22 tex (soit approximativement 98±11 deniers) et le second monofilament (16) présente une masse linéique d'approximativement 26,67 tex ± 2,22 tex (soit approximativement 240±20 deniers) ; ou
facultativement les premier et second monofilaments (12, 16) présentent une ténacité d'approximativement 5,4 à 7,65 grammes par dtex (approximativement 6,00 à 8,50 grammes par denier) ; ou
facultativement les premier et second monofilaments (12, 16) présentent une ténacité d'approximativement 5,58 grammes par dtex (approximativement 6,2 grammes par denier).

3. Tissu de réparation prothétique implantable selon la revendication 1, dans lequel la maille (10) présente une construction de tricot qui inclut approximativement 34 à 36 rangées de mailles par 25,4 mm (34 à 36 rangées de mailles par pouce) et approximativement 12 à 17 colonnes de mailles par 25,4 mm (12 à 17 colonnes de mailles par pouce) ; ou
dans lequel la maille (10) présente un poids par unité de surface d'approximativement 0,0102 à 0,0107 grammes/cm² (0,0661 à 0,0690 grammes par pouce carré) ; ou
dans lequel la maille (10) présente une épaisseur d'approximativement 0,589 à 0,610 mm (0,022 à 0,024 pouces).

4. Tissu de réparation prothétique implantable selon la revendication 1, dans lequel la maille (10) présente une résistance à l'éclatement à la bille de 15,88 kg à 19,23 kg (35 lbs à 42,4 lbs) ;
facultativement dans lequel la maille (10) présente une résistance à l'éclatement à la bille de 16,33 kg à 18,14 kg (36 lbs à 40 lbs) ; ou
facultativement dans lequel la maille (10) présente une résistance à l'éclatement à la bille de 16,33 kg à 17,69 kg (36 lbs à 39 lbs).

5. Tissu de réparation prothétique implantable selon la revendication 1, dans lequel la maille (10) présente une résistance à l'arrachement de suture de 4,08 kg à 4,99 kg (9 lbs à 11 lbs) dans le sens machine et de 3,40 kg à 4,31 kg (7,5 lbs à 9,5 lbs) dans le sens travers ;
facultativement dans lequel la maille (10) présente une résistance à l'arrachement de suture d'approximativement 4,63 kg (10,2 lbs) dans le sens machine et d'approximativement 3,86 kg (8,5 lbs) dans le sens travers.

6. Tissu de réparation prothétique implantable selon la revendication 1, dans lequel la maille (10) présente une résistance à la traction de 5,35 kg à 7,62 kg (11,8 lbs à 16,8 lbs) dans le sens machine et de 15,51 kg à 21,41 kg (34,2 lbs à 47,2 lbs) dans le sens travers ;
facultativement dans lequel la maille (10) présente une résistance à la traction d'approximativement 6,49 kg (14,3 lbs) dans le sens machine et d'approximativement 18,46 kg (40,7 lbs) dans le sens travers.

7. Tissu de réparation prothétique implantable selon la revendication 1,
dans lequel la maille (10) présente une résistance à l'éclatement à la bille de 15,88 kg à 19,23 kg (35 lbs à 42,4 lbs), une résistance à l'arrachement de suture de 4,08 kg à 4,99 kg (9 lbs à 11 lbs) dans le sens machine et de 3,40 kg à 4,31 kg (7,5 lbs à 9,5 lbs) dans le sens travers, et une résistance à la traction de 5,35 kg à 7,62 kg (11,8 lbs à 16,8 lbs) dans le sens machine et de 15,51 kg à 21,41 kg (34,2 lbs à 47,2 lbs) dans le sens travers.

8. Tissu de réparation prothétique implantable selon la revendication 7, dans lequel la maille (10) présente une résistance à l'éclatement à la bille de 16,33 kg à 17,69 kg (36 lbs à 39 lbs).

9. Tissu de réparation prothétique implantable selon la revendication 7, dans lequel la maille (10) présente une résistance à l'arrachement de suture d'approximativement 4,63 kg (10,2 lbs) dans le sens machine et d'approximativement 3,86 kg (8,5 lbs) dans le sens travers.

10. Tissu de réparation prothétique implantable selon la revendication 7, dans lequel la maille (10) présente une résistance à la traction d'approximativement 6,49 kg (14,3 lbs) dans le sens machine et d'approximativement 18,46 kg (40,7 lbs) dans le sens travers.

11. Tissu de réparation prothétique implantable selon la revendication 7, dans lequel la maille (10) présente une construction de tricot qui inclut approximativement 34 à 36 rangées de mailles par 25,4 mm (34 à 36 rangées de mailles par pouce) et approximativement 12 à 17 colonnes de mailles par 25,4 mm (12 à 17 colonnes de mailles par pouce).

12. Tissu de réparation prothétique implantable selon la revendication 7, dans lequel la maille (10) présente un poids par unité de surface d'approximativement 0,0102 à 0,0107 grammes/cm² (0,0661 à 0,0690 grammes par pouce carré).

13. Tissu de réparation prothétique implantable selon la revendication 7, dans lequel la maille (10) présente une épaisseur d'approximativement 0,589 à 0,610 mm (0,022 à 0,024 pouces).
